# EUROPÄISCHE PATENTSCHRIFT

(11) **EP 3 964 165 B1**
(45) Veröffentlichungstag und Bekanntmachung des Hinweises auf die Patenterteilung: **26.03.2025**
(21) Anmeldenummer: 20194142.4
(22) Anmeldetag: 02.09.2020
(51) Int. Cl.: A61C 13/10, A61C 13/093, A61C 13/103, A61C 13/01, A61C 13/09, A61C 13/277

(54) **DENTALPROTHESE**
DENTAL PROSTHESIS
PROTHÈSE DENTAIRE

(43) Veröffentlichungstag der Anmeldung: 09.03.2022
(73) Patentinhaber: Ivoclar Vivadent AG, 9494 Schaan (LI)
(72) Erfinder: BAASKE, Thomas, 8885 Mols (CH); FERILLI, Antonio, 9545 Wängi (CH)
(74) Vertreter: Baldus, Oliver

(56) Entgegenhaltungen:
- DE-A1- 102009 056 752
- US-A- 4 059 901
- US-A1- 2018 280 123

## Beschreibung

Die Erfindung betrifft eine Dentalprothese, gemäß dem Oberbegriff von Anspruch 1.

Dentalprothesen bestehen typischerweise aus einer fleischfarbenen Prothesenbasis und einer zahnfarbenen Zahnanordnung. Diese sind fest miteinander verbunden, wobei die Prothesenbasis die Zahnanordnung fasst und hält, und wobei die Zahnanordnung typischerweise in die Prothesenbasis teilweise eingebettet ist. Zudem dient die Prothesenbasis der Auflage und Verankerung auf bzw. an dem Kieferkamm.

Durch die Kaubewegungen und die hiermit einhergehenden Wechsellasten sind Dentalprothesen gerade an ihrer Auflage bzw. Verankerung besonderen Belastungen ausgesetzt.

Typischerweise werden mindestens zwei Implantate im Kieferknochen verankert, und es wird mittels sogenannter Primärstrukturen und Sekundärstrukturen eine Verankerung sichergestellt.

Die Verankerung erfolgt zwischen einem Implantat, gegebenenfalls mit Abutment, mit einer Patrize, die auch als Primärstruktur oder als Innenteleskop bezeichnet wird, und einer an der Dentalprothese angebrachten Matrize, die auch als Sekundärstruktur oder Außenteleskop bezeichnet wird.

Nachdem diese Verankerung erhebliche Kräfte zur Prothese hin übertragen muss, wird ein typischerweise metallisches Verstärkungsgerüst als Tertiärstruktur eingesetzt, welches die Sekundärstruktur umgibt und für die bessere Kraftverteilung in die Prothese und zu der Zahnanordnung hin sorgen soll.

Es ist auch vorgeschlagen worden, die Zahnanordnung, gerade wenn sie einzelne Zähne aufweist, an der Tertiärstruktur zu verankern. Diese Lösung ist jedoch vergleichsweise aufwändig und erfordert es, dass die Höhenlage der Zähne vorab genau festliegt.

Ferner sind als Sonderform von Dentalprothesen sogenannte Hybridprothesen bekannt geworden.

Diese ermöglichen eine bessere Kraftverteilung. Abgesehen von der Verankerung an Primär-, Sekundär- und Tertiärstrukturen über die Implantate weist bei einer Hybridprothese die Prothesenbasis mindestens zwei Auflageflächen auf.

Diese sind genau angepasst an den Kieferkamm des Patienten ausgelegt und in der Lage, schmerzfrei erhebliche Kräfte aufzunehmen.

Bei einem zahnfreien Mund können sich mehrere Auflageflächen verteilt über den gesamten Kieferkamm erstrecken, oder auch eine große Auflagefläche insgesamt bilden. Diese Auflagefläche kann nach der Art eines Zahnbogens in Form eines im Querschnitt U-förmigen Kanals ausgebildet sein, so dass sie nicht nur okklusale, sondern auch schräg auftreffende und horizontale Kräfte aus der Kaubewegung aufnehmen kann.

Derartige Hybridprothesen sind beispielsweise aus der FR 2 593 058 A1 und der US 7 657 510 A1 bekannt. Bei der genannten US 7 657 510 A1 wird jeder Zahn mittels eines speziellen Implantatsystems abgestützt. Dieses bietet zwar eine gute Verankerung, ist jedoch nicht immer anwendbar und erfordert auch eine Mehrzahl von Kieferknochen-Bohrungen.

Bei der Lösung gemäß der FR 2 593 058 A1 soll eine gute Verankerung zwischen der Prothese und der Primärstruktur realisiert sein, und die Austauschbarkeit soll gegeben sein.

Ferner ist es vorgeschlagen worden, Hybridprothesen dergestalt auszubilden, dass eine beschränkte Anzahl von Primärstrukturen als Implantataufbauteil, die auf Implantaten abgestützt sind, beispielsweise 2, 3 oder 4, im Bereich der Schneidezähne und Prämolaren oder gegebenenfalls je eine im Bereich der Molaren realisiert ist, und distal vom hintersten Implantat aus gesehen eine vergrößerte Auflagefläche realisiert ist, die der dort verbesserten Abstützung diesen soll. Diese Lösung bietet einen guten Kompromiss hinsichtlich der Aufnahme und Verteilung der Kaukräfte. Sie ist jedoch im Hinblick auf die aufwändig einzubettende Tertiärstruktur vergleichsweise teuer und unflexibel.

US 2018/280123 A1 offenbart eine Vollbogen- oder Teilbogenprothese, die einen schraubenaufnehmenden Zylinder aufweist.

Demgegenüber liegt der Erfindung die Aufgabe zugrunde, eine Dentalprothese gemäß dem Oberbegriff von Anspruch 1 zu schaffen, die flexibel handhabbar ist und bei gleichen oder geringeren Herstellkosten eine mindestens gleich gute Dauerhaltbarkeit gepaart mit einem gute Tragekomfort bietet.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, die Zahnanordnung als einstückiger Vollzahnbogen oder als segmentierter Zahnbogen zu realisieren.

Ein solcher segmentierter Zahnbogen, der auch als Zahnbogen-Segment bezeichnet werden kann, ermöglicht zum einen in effizienter Weise eine Fertigung per CAD/CAM. Zum anderen hat es sich gezeigt, dass eine Dentalprothese, die aus segmentierten oder einstückigen Zahnbögen anstelle von Einzelzähnen aufgebaut ist bzw. diese aufweist, eine erheblich verbesserte Biegefestigkeit und auch Biegezugfestigkeit aufweist.

Im Vergleich mit der Realisierung der Prothesen mit Einzelzähnen kann die Biegefestigkeit doppelt so groß sein.

Erfindungsgemäß bevorzugt ist der Zahnbogen aus einem härteren Material als die Prothesenbasis, was die vorstehende Eigenschaft gewährleistet.

Erfindungsgemäß ist es vorgesehen, dass die Zahnanordnung als Zahnbogen im Vergleich mit den bislang bekannten Hybridprothesen nach gingival verlängert ist. Der Zahnbogen bildet an seiner basalen Seite ein Lagerelement für die Lagerung der Prothese.

Das Lagerelement nimmt ein Sekundärteil auf und umgibt dieses. Das Sekundärteil kann z.B. als Außenteleskop ausgebildet sein.

In an sich bekannter Weise interagiert das Sekundärteil mit einem Primärteil. Das Primärteil kann beispielsweise durch ein Abutment oder einen Zahnstumpf gebildet sein, oder aber je durch ein Teil von diesem gebildet sein.

Bevorzugt ist der erfindungsgemäße Zahnbogen nach gingival verlängert. Die Zahnbogenverlängerung nach gingival simuliert erfindungsgemäß eine Tertiärstruktur.

Die Tertiärstruktur ist erfindungsgemäß entbehrlich. Die erfindungsgemäße Prothese ist frei von Tertiärstrukturen.

Damit ist aber auch sichergestellt, dass keine Verstärkungselemente, bspw. metallischen Elemente, in die Prothesenbasis eingebettet werden müssen.

Die Prothesenbasis lässt sich daher ebenfalls leichter per Fräsen herstellen. Besonders günstig ist es, wenn die Zahnanordnung nach gingival bis zur Sekundärstruktur reicht, diese umgibt und aufnimmt und damit der Verankerung und der Übertragung von Kaukräften dient. Sie hat dann direkten Kontakt mit der Sekundärstruktur, oder Kontakt über eine dünne Schicht Basismaterial.

Bevorzugt ist der Kontakt nicht lediglich eine Anlage, sondern ein mindestens teilweise Umgreifen. Um einen solchen umgreifenden Kontakt zu gewährleisten, ist die Zahnanordnung oder der Zahnbogen an ihrer gingivalen bzw. basalen Seite kanalförmig, entsprechend dem Verlauf des Zahnbogens, oder weist zumindest geeignete passende Ausnehmungen an den Stellen auf, an denen eine Sekundärstruktur eingebracht werden soll. Die Zahnanordnung bildet dort, also an ihrer basalen Seite, ein Lagerelement zur Lagerung der Sekundärstruktur.

Diese ist in geeigneter Weise mit dem Lagerelement verbunden. Diese Verbindung kann durch Einpolymerisieren oder durch einen Kleber realisiert sein.

In vorteilhafter Ausgestaltung hat der Zahnbogen an seiner unteren, also basalen oder gingivalen Seite unmittelbaren Kontakt mit der Sekundärstruktur. Dies ermöglicht es eine sichere Verankerung und gute Kraftübertragung. Dies gilt insbesondere dann, wenn das Sekundärteil vollständig in dem unteren Bereich des oder Basalbereich des Zahnbogens aufgenommen ist. Sekundärteil und Sekundärstruktur werden hier synonym verwendet.

In modifizierter Ausgestaltung ist es vorgesehen, dass der Zahnbogen in seinem Basalbereich geeignet geformt ist, um die Sekundärstruktur aufzunehmen. Der Zahnbogen ist bei dieser Ausführungsform von einer dünnen Schicht von Basismaterial abgedeckt, die ihrerseits das Sekundärteil aufnimmt. In diesem Fall ist bevorzugt ein Kleber vorgesehen, der die Sekundärstruktur dort fixiert.

Diese Lösung ermöglicht eine etwas elastischere Aufnahme des Sekundärteils in dem Zahnbogen, ohne dass die Aufnahme gerade auch von Scherkräften beeinträchtigt wäre.

In den bevorzugten Ausführungsformen der Erfindung sind die Zahnsegmente des Zahnbogens oder der Zahnbogen so gestaltet, dass pro Segment oder pro Zahnbogen mindestens zwei Sekundärteile der Verankerung dienen. Dies ermöglicht eine besonders biegesteife und verbindungssteife Verankerung des Zahnbogens am Kieferknochen.

Bevorzugt ist der Zahnbogen oder das Zahnsegment so gestaltet, dass an den Stellen, an denen Sekundärstrukturen vorgesehen sind, im Basalbereich entsprechende Ausnehmungen vorliegen.

Der Basalbereich im Übrigen ist mit dem Zahnbogenmaterial gefüllt und von Prothesenbasismaterial dort abgedeckt.

In modifizierter Ausgestaltung ist ein durchlaufender Kanal vorgesehen, der an den Stellen, an denen die Sekundärstruktur vorgesehen ist, mit dieser gefüllt ist. Demgegenüber ist der Kanal dann an den Stellen außerhalb der Sekundärstrukturen mit Prothesenbasismaterialien gefüllt.

Während hier die erfindungsgemäße Dentalprothese in Verbindung mit einer Teleskoplagerung mit einem Innenteleskop = Primärteil und einem Außenteleskop = Sekundärteil beschrieben ist, versteht es sich, dass anstelle dessen auch eine Lagerung auf einem Abutment, das seinerseits auf dem Implantat verankert ist, möglich ist. Alternativ ist auch die Lagerung auf einem Zahnstumpf möglich.

Das Abutment erstreckt sich dann an der Stelle, an der das Sekundärteil vorgesehen ist, und es ist in gleicher Weise eine sichere Lagerung und Verankerung am Zahnbogenmaterial möglich, gegebenenfalls über eine dünne Schicht von Prothesenbasismaterial.

Die erforderlichen Ausnehmungen lassen sich im Zahnbogenmaterial ohne weiteres realisieren, da die Dentalprothese ohnehin bevorzugt durch abtragende Verfahren wie durch Fräsen oder aufbauenden Verfahren gefertigt ist.

Hinsichtlich der Verbindung zwischen Prothesenbasis und Zahnanordnung ist es bevorzugt, dass beide Elemente aus separaten Rohlingen gefertigt sind. Der Zahnbogen wird an den Kontaktstellen zur Prothesenbasis in an sich bekannter Weise mit einem selbst polymerisierenden Kleber versehen, in die Zahnbogenausnehmung der Prothesenbasis eingesetzt und polymerisiert dann selbsttätig.

In gleicher Weise lässt sich nach erfolgter Verbindung zwischen Prothesenbasis und Zahnanordnung die Sekundärstruktur in die hierfür vorgesehene Ausnehmung oder den hierfür vorgesehenen Kanal einsetzen, wobei mit einem entsprechenden Kleber für eine gute Haftung gesorgt ist.

Es ist auch möglich, die Sekundärstruktur mit kleinen Ausnehmungen zu versehen, und entsprechende Vorsprünge innen an dem Lagerelement der Zahnanordnung zu realisieren. Beim Aufschieben schnappen dann die Vorsprünge in die Ausnehmungen ein, was die Fixierung und Lagerung der Zahnanordnung auf der Sekundärstruktur noch verbessert. Insofern ist auch eine rein formschlüssige Lagerung, oder ggf. - mittels Kleber - eine kombinierte Klebe- und Formschlussverbindung möglich.

Es ist auch möglich, bei der Realisierung einer Schnapprastverbindung zwischen Sekundärteil und der zugehörigen Ausnehmung in der Zahnanordnung zu realisieren und dann zunächst die Sekundärstruktur dort einzurasten.

Die unteren Flügel des Basalbereichs des Zahnbogens sind vergleichsweise nachgiebig, was das Einrasten erleichtert. Bei dieser Lösung ist es vorgesehen, erst anschließend hieran die Kombination aus Sekundärteil und Zahnbogen in das Prothesenbasismaterial einzukleben und damit eine Aussteifung bereitzustellen.

Erfindungsgemäß ist es vorgesehen, vollständig auf eine Tertiärstruktur zu verzichten. Eine Verstärkungsstruktur, bspw. Metalleinlagerung, in die Prothesenbasis ist daher nicht erforderlich, und die hierfür erforderlichen aufwendigen Schritte können entfallen.

Wenn mindestens die Zahnanordnung per CAD/CAM, also in der Regel per Fräsen, hergestellt wird, bietet es sich an, eine virtuelle Form des Basalbereichs der Zahnanordnung zu hinterlegen, die dann stets zu der zugehörigen Sekundärstruktur passt.

Bevorzugt sind hier kantenfreie Übergänge. Bei Durchmesseranpassungen zwischen der Zahnwurzel und dem Basalbereich des Zahns, der durch die U-förmige oder kanalförmige Ausgestaltung gekennzeichnet ist, ist es bevorzugt, weiche Übergänge zu realisieren, also beispielsweise S-Formen.

Es versteht sich, dass aber auch anstelle dessen kurzerhand die Zahnwurzel so verbreitet oder schmaler gehalten wird, dass sie zu dem U-förmigen Basalbereich des erfindungsgemäßen Zahnbogens passt.

Ferner ist es auch möglich, den Anschluss des Basalbereichs an den Kieferkamm beliebig zu gestalten. Wenn in der ersten Ausführungsform der Basalbereich des Zahns aus den härteren Zahnbogenmaterial unmittelbar an der Sekundärstruktur anliegt, kann aber dennoch der Teil des Basalbereichs, der zum Kieferkamm hin gewandt ist, von einer dünnen Schicht aus Prothesenbasismaterial geschützt werden. Diese Lösung ist bevorzugt.

Alternativ ist es auch möglich, das Zahnbogenmaterial dort so weit herunterzuziehen, dass ein Kontakt zwischen dem Kieferkamm und dem Zahnbogenmaterial entsteht.

Es ist auch möglich, das Zahnbogenmaterial vor dem Kieferkamm enden zu lassen, so dass eine Schicht aus Basismaterial sich zwischen dem Kieferkamm und dem Zahnbogen erstreckt, und das Prothesenbasismaterial den Kieferkamm vollständig abdeckt.

**In** vorteilhafter Ausgestaltung ist es vorgesehen, dass die Zahnanordnung durch den vollständigen Zahnbogen gebildet ist. Bei dieser Ausgestaltung ist die Zahnanordnung besonders biegefest, nachdem das Zahnmaterial eine hohe Biegefestigkeit aufweist.

Es versteht sich, dass bei Bedarf die Zahnanordnung im Basalbereich außerhalb der Stellen, an denen die Sekundärstrukturen und die Auflageflächen vorgesehen sind, wie beliebige geeignete Ausgestaltungen haben können. Beispielsweise ist es möglich, zur Erhöhung der Steifigkeit Rippen oder Vorsprünge zu realisieren, die die Biegefestigkeit und Biegezugfestigkeit des Zahnbogens an sich, und damit auch insgesamt der Dentalprothese erhöhen können.

**In** einer modifizierten Ausgestaltung ist es vorgesehen, den Basalbereich des Zahnbogens an den hier angesprochenen Stellen mit einer Art Rippen/Gitterstruktur zu versehen und den Zahnbogen durch ein abtragendes Verfahren wie durch Fräsen auf einer CAD/CAM-Vorrichtung herzustellen, und die Prothesenbasis durch ein auftragendes Verfahren wie beispielsweise per Rapid Prototyping direkt an den Zahnbogen an der geeigneten Stelle anzuformen.

Eine entsprechend hochwertige Dentalprothese lässt sich auch per Spritzguss anspritzen, wobei die Spritzgussform dann bevorzugt ebenfalls per Rapid Prototyping hergestellt ist.

Die Art und Anordnung der Sekundärstrukturen und der Auflageflächen lässt sich in weiten Bereichen an die Erfordernisse anpassen. So können beispielsweise je eine Sekundärstruktur im Eckzahnbereich und eine Auflagefläche am distalen Ende der Prothese bereitgestellt werden.

Diese Lösung hat den Vorteil einer statisch bestimmten Fixierung der Dentalprothese auf dem Kieferkamm, und zugleich den Vorteil einer schonenden Kraftverteilung auf großen Auflageflächen, die dem Tragekomfort erhöhen.

Bei Realisierung der Zahnanordnung aus Segmenten des gesamten Zahnbogens, also aus Zahnteilbögen, ist es auch möglich, Auflageflächen im Übergangsbereich zwischen Segmentteilbögen zu realisieren. Bei dieser Lösung ist dann bevorzugt für jedes Zahnsegment eine Sekundärstruktur vorgesehen, die der Verankerung dient. Es entsteht so eine abwechselnde Krafteinleitung über Sekundärstrukturen in den Kieferknochen des Patienten und über Auflagefläche auf dem Kieferkamm.

Erfindungsgemäß wesentlich ist es, dass vollständig auf metallische Einlagerungen oder Anlagerungen der Hybridprothese verzichtet werden kann. Es muss keine Tertiärstruktur gefertigt oder eingefügt oder angefügt werden, um die erwünschte Kraftübertragung und Stabilität bereitzustellen.

Weitere Einzelheiten, Vorteile und Merkmale ergeben sich aus der nachfolgenden Beschreibung mehrerer Ausführungsbeispiele der Erfindung anhand der Zeichnung.

Es zeigen:
- Fig. 1: eine schematische perspektivische Ansicht einer Dentalprothese als Hybridprothese in einer Ausführungsform der Erfindung;
- Fig. 2: eine Schnittansicht durch eine nicht erfindungsgemäße Dentalprothese in einer weiteren Ausführungsform;
- Fig. 3: eine Schnittansicht durch eine Dentalprothese in einer weiteren Ausführungsform;
- Fig. 4: eine Schnittansicht durch eine Dentalprothese in einer weiteren Ausführungsform;
- Fig. 5: eine Schnittansicht durch eine Dentalprothese in einer weiteren Ausführungsform;
- Fig. 6: eine Schnittansicht durch eine nicht erfindungsgemäße Dentalprothese in einer weiteren Ausführungsform; und
- Fig. 7: eine Schnittansicht durch eine nicht erfindungsgemäße Dentalprothese in einer weiteren Ausführungsform.

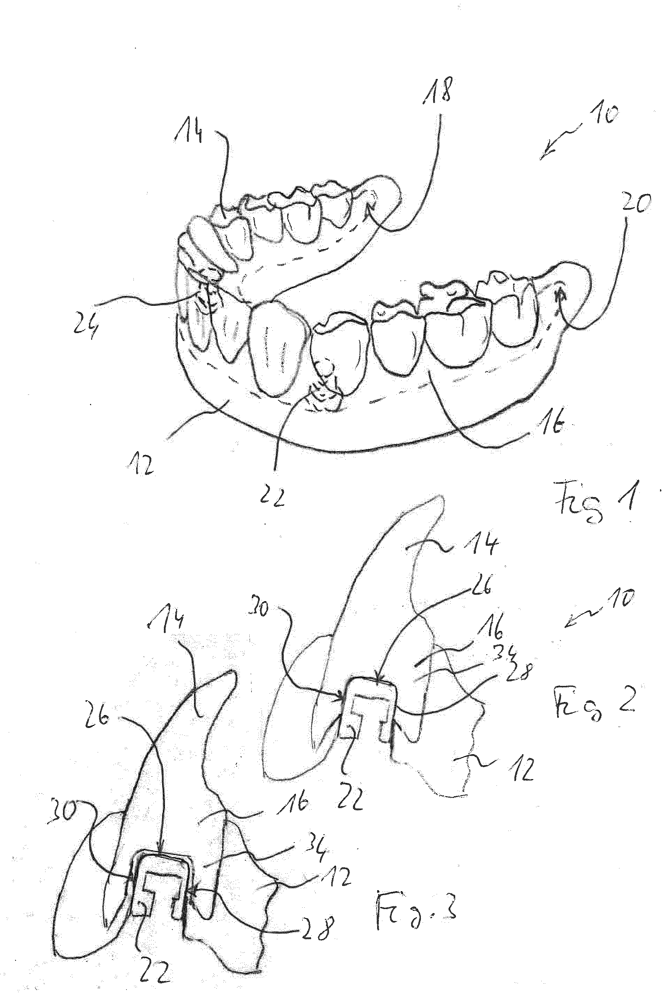

Die In Fig. 1 dargestellte Dentalprothese 10 besteht in an sich bekannter Weise bei sämtlichen Ausführungsformen der Erfindung aus einer Prothesenbasis 12 und einer Zahnanordnung 14, wobei in ebenfalls an sich bekannter Weise die Prothesenbasis 12 fleischfarben und die Zahnanordnung 14 zahnfarben ist.

Die Zahnanordnung 14 ist auf einer gemeinsamen Zahnbasis 34 als Zahnbogen 16 aufgebaut, in dem dargestellten Ausführungsbeispiel einstückig.

Die Prothesenbasis 12 nimmt die Zahnbasis vollständig auf, wobei in an sich bekannter Weise das Prothesenbasismaterial 12 im Bereich der Zahnübergänge hochgezogen ist, was die Anmutung von Einzelzähnen ermöglicht.

Der Zahnbogen 16 besteht aus einem härteren Material als die Prothesenbasis 12, beispielsweise hoch vernetztem PMMA.

Die Prothesenbasis 12 weist zwei Auflageflächen 18 und 20 auf, die mit ihrer Flächennormalen nach unten weisen und dafür bestimmt sind, auf dem Kieferkamm 42 des Patienten aufzuliegen. Diese sind bevorzugt am distalen Ende der Prothese 10 ausgebildet.

Hier ist unter Flächennormale eine geeignete Flächennormale zu verstehen, die nach unten weist, so dass die Fläche als Auflagefläche 18 dienen kann. Tatsächlich sind die Auflageflächen 18 und 20 gekrümmt, entsprechend dem an dieser Stelle geformten Kieferkamm 42.

Für die Verankerung der Dentalprothese 10 am Kiefer des Patienten sind ferner zwei schematisch dargestellte Sekundärstrukturen 22 und 24 vorgesehen. Diese sind in an sich bekannter Weise als Außenteleskop oder Matrize ausgebildet und ruhen auf einer nicht dargestellten Primärstruktur 38 oder einem Innenteleskop, die ihrerseits auf einem Implantat 40 im Kieferknochen des Patienten verankert ist.

Die Zahnbasis 34, also der von dem Basismaterial verdeckte Bereich des Zahnbogens 16, umgibt die Sekundärstrukturen 22 und 24. Sie reicht nach unten über die Sekundärstrukturen 22 und 24 hinaus und bildet ein Lagerelement für die Aufnahme der Sekundärstrukturen 22 und 24. Beispielhafte Ausgestaltungen hierfür sind aus den Fig. 2 bis 7 ersichtlich.

In Fig. 2 ist eine Sekundärstruktur 22 dargestellt, die an drei Seiten, also an der Okklusalseite 26, der Oralseite 28 und der Vestibulärseite 30 von Zahnmaterial umgeben und umschlossen ist. Das Zahnmaterial liegt großflächig an der Sekundärstruktur 22 an. Die Sekundärstruktur 22 ist in an sich bekannter Weise an dieser Stelle in die Dentalprothese 10 eingeklebt, und die eingeleiteten Kräfte werden vollständig oder nahezu vollständig von dem Zahnbogen 16 aufgenommen. Insofern bildet der Zahnbogen 16 an dieser Stelle die Form eines nach unten offenen U.

Die gingivalen Seitenschenkel der Sekundärstruktur 22 sind jedoch von dem Material der Prothesenbasis 12 abgedeckt. Dieses Material hat eine größere Elastizität und ist weicher, so dass es besser für die Anlage an dem Kieferkamm geeignet ist. Es umgibt den Bereich der Zahnbasis 34 vollständig.

Eine modifizierte Ausgestaltung einer erfindungsgemäßen Dentalprothese 10 im Schnitt ist in Fig. 3 dargestellt. Hier wie auch in den weiteren Figuren weisen gleiche Bezugszeichen auf gleiche oder entsprechende Teile hin. Im Unterschied zu der Lösung gemäß Fig. 2 ist dort die Sekundärstruktur 22 von einer dünnen Schicht Basismaterial 12 abgedeckt.

Diese ist von der Zahnbasis 34 aufgenommen, die insofern über die Schicht Basismaterial 12 wiederum die Okklusalseite 26, die Oralseite 28 und die Vestibulärseite 30 der Sekundärstruktur 22 abstützt.

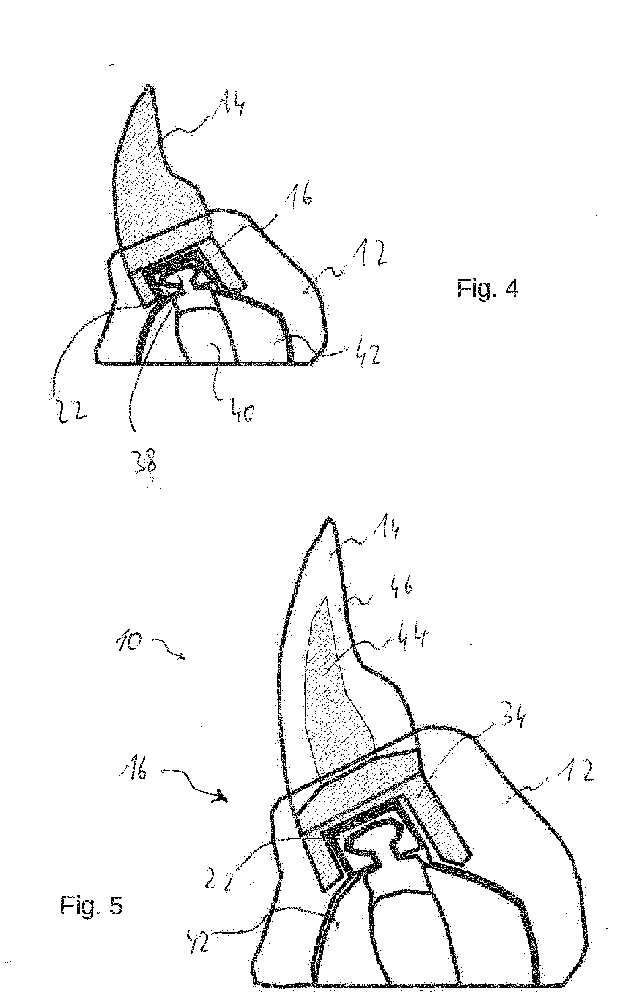

Fig. 4 zeigt eine modifizierte Ausgestaltung der Dentalprothese 10, wobei neben der Sekundärstruktur 22 auch eine Primärstruktur 38 dargestellt ist.

Die Primärstruktur 38 ist über ein Implantat 40 im Kieferknochen verankert, welches vom Kieferkamm 42 aufgenommen ist. Auf dem Kieferkamm 42 liegt das Prothesenbasismaterial 12 flächig auf, und ermöglicht so eine kippsichere Lagerungen der Prothese 10.

Auch bei der Ausführungsform gemäß Fig. 4 reicht das Prothesenbasismaterial 12 bis unter die Zahnbasis 34 und deckt die Sekundärstruktur 22 ab.

Aus Fig. 5 ist eine weitere Ausgestaltung der erfindungsgemäßen Dentalprothese 10 ersichtlich. Bei dieser Lösung ist die Zahnanordnung 14 zweischichtig ausgebildet und besteht aus einer Dentinschicht 44 und einer Zahnschmelzschicht 46.

Die Dentinschicht 44 ist in an sich bekannter Weise im Inzisalbereich, als außerhalb der Prothesenbasis 12, vollständig von dem Zahnschmelz 46 abgedeckt. Die Dentinschicht 44 erstreckt sich aber in Form einer Verlängerung über die Sekundärstruktur 22.

Insofern erstreckt sich auch die Zahnanordnung 14 im Bereich der Zahnbasis 34 über die Sekundärstruktur 22. Sie stützt den Zahnbogen 16 an dieser Stelle ab und damit die Dentalprothese 10.

In an sich bekannter Weise ist die Dentinschicht 44 opaker und das Schmelzmaterial 46 durchscheinender. Auch hier ist der Bereich des Zahnbogens 16 vollständig vom Prothesenbasismaterial 12 abgedeckt.

Anstelle einer Lagerung auf einer Sekundärstruktur 22, die mit einer Primärstruktur 38 zusammenwirkt, ist es auch möglich, eine Lagerung der Prothesenbasis 12 und damit des Zahnbogens 16 auf einem Abutment 50 zu realisieren. Das Abutment 50 ist wiederum in an sich bekannter Weise auf dem Implantat 40 gelagert.

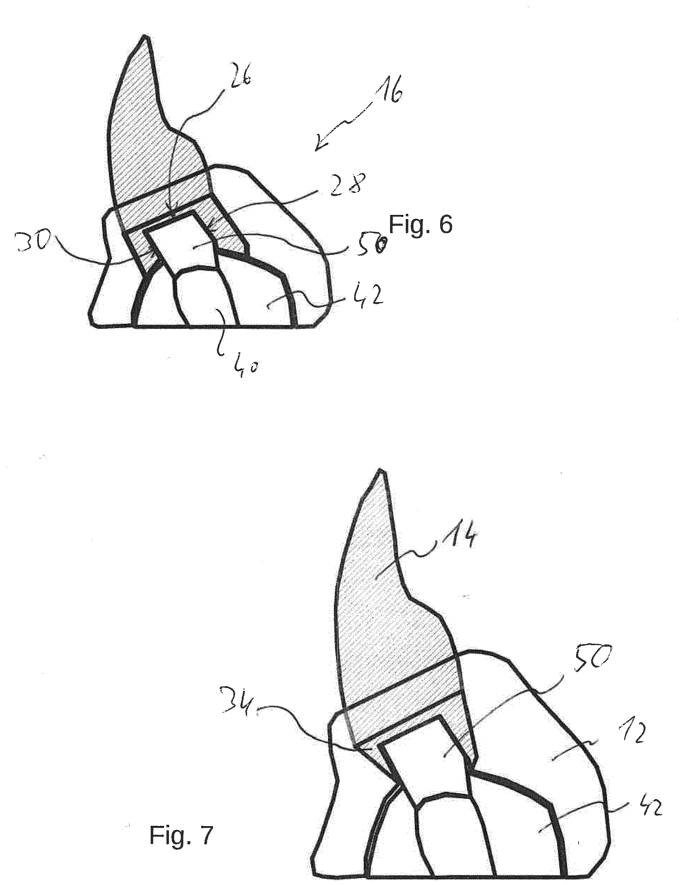

Diese Lösung ist in Fig. 6 dargestellt. Die Prothesenbasis 12 umschließt wiederum an den drei Seiten 26, 28 und 30 das Abutment 50. Auch hier ist es nicht erforderlich, eine zusätzliche Stützstruktur in Form einer Tertiärstruktur zu realisieren.

Aus Fig. 7 ist eine weitere Ausgestaltung einer Ausführungsform mit dem Implantat 40 dargestellt. Bei dieser Lösung verläuft der Zahnbogen 16 mit der Zahnbasis 34 in direktem Kontakt dem Implantat 40 und ist nach unten hin spitz zulaufend. Dies verhindert im Gegensatz zu der Ausführungsform gemäß Fig. 6 einen direkten Kontakt zwischen dem Kieferkamm 42 und dem Zahnbogen 16.

Auch bei dieser Lösung ist die seitliche und damit den Kaubewegungen Rechnung tragende Abstützung ohne weiteres gegeben.

## Patentansprüche

1. Dentalprothese, mit einer fleischfarbenen Prothesenbasis (12) und einer zahnfarbenen Zahnanordnung (14) die in die Prothesenbasis teilweise eingebettet ist, wobei die Dentalprothese als Hybridprothese ausgebildet ist, also wobei die Prothesenbasis eine Auflagefläche aufweist, die eine nach gingival weisende Flächennormale hat, wobei die Zahnanordnung (14) ein Sekundärteil (22) oder ein Primärteil (50) an mindestens zwei Seiten (26, 28, 30) umgibt und als einstückiger Zahnbogen oder als segmentierter Zahnbogen (16) ausgebildet ist **dadurch gekennzeichnet, dass** das Sekundärteil oder das Primärteil die Zahnanordnung abstützt und zwischen Zahnanordnung und Sekundärteil oder Primärteil eine Schicht von Prothesenmaterial der Prothesenbasis liegt.

2. Dentalprothese nach Anspruch 1, **dadurch gekennzeichnet, dass** das zahnfarbene Zahnmaterial der Zahnanordnung aus einem härteren Material als das Prothesenmaterial der Prothesenbasis (12) besteht.

3. Dentalprothese nach einem der vorhergehenden Ansprüche, **dadurch gekennzeichnet, dass** das Sekundärteil als Außenteleskop ausgebildet ist und/oder, dass das Primärteil als Abutment oder als Zahnstumpf ausgebildet ist.

4. Dentalprothese nach einem der vorhergehenden Ansprüche, **dadurch gekennzeichnet, dass** die Zahnanordnung (14) durch ein abtragendes oder aufbauendes Verfahren hergestellt ist, insbesondere aus dem Vollen gefräst ist.

5. Dentalprothese nach einem der vorhergehenden Ansprüche, **dadurch gekennzeichnet, dass** die Zahnanordnung (14) an ihrer basalen Seite das Außenteleskop oder das Primärteil oral, vestibulär und okklusal umgibt.

6. Dentalprothese nach einem der vorhergehenden Ansprüche, **dadurch gekennzeichnet, dass** die Zahnanordnung (14) an ihrer basalen und damit gingivalen Seite ein Lagerelement ausbildet, das das Sekundärteil oder das Primärteil aufnimmt, ggf. über eine Schicht Prothesenmaterial der Prothesenbasis.

7. Dentalprothese nach Anspruch 6, **dadurch gekennzeichnet, dass** das Lagerelement der Zahnanordnung nach außen hin, also vestibulär und oral, in Prothesenmaterial der Prothesenbasis (12) eingebettet ist, welches das Lagerelement von außen abstützt, und dass das Lagerelement innen nach gingival freiliegt, also offen ist.

8. Dentalprothese nach einem der vorhergehenden Ansprüche, **dadurch gekennzeichnet, dass** die Zahnanordnung (14) mindestens teilweise aus hochvernetztem PMMA, insbesondere aus DCL-Material, besteht, und die Prothesenbasis (12) aus weniger hoch vernetztem PMMA.

9. Dentalprothese nach Anspruch 6, **dadurch gekennzeichnet, dass** die Zahnanordnung (14) zweischichtig oder mehrschichtig aus Dentinmaterial und aus Schmelzmaterial aufgebaut ist, welches durchscheinender als das Dentinmaterial ist, und dass das Dentinmaterial sich einstückig zum Lagerelementerstreckt und dieses ausbildet.

10. Dentalprothese nach Anspruch 6, **dadurch gekennzeichnet, dass** die Zahnanordnung (14) Segmente aus mindestens 3 Zähnen aufweist, die über Dentinmaterial miteinander und mit dem Lagerelement fest verbunden sind.

11. Dentalprothese nach einem der vorhergehenden Ansprüche, **dadurch gekennzeichnet, dass** die Dentalprothese (10) metallfrei ist und per CAD/CAM, insbesondere in abtragenden oder aufbauenden Verfahren, hergestellt ist.

12. Dentalprothese nach Anspruch 6, **dadurch gekennzeichnet, dass** das Lagerelement aus Zahnmaterial in der Draufsicht bogenförmig, entsprechend dem Zahnbogen (16), ausgebildet ist, und im Schnitt U-förmig, für die Unterkieferprothese in Form eines auf dem Kopf stehenden U.

13. Dentalprothese nach einem der vorhergehenden Ansprüche, **dadurch gekennzeichnet, dass** das Zahnmaterial sich bis zur Auflagefläche erstreckt und von diese bildendem Prothesenmaterial der Prothesenbasis abgedeckt ist.

14. Dentalprothese nach einem der vorhergehenden Ansprüche, **dadurch gekennzeichnet, dass** das Zahnmaterial von einer Schicht von Prothesenmaterial der Prothesenbasis abgedeckt ist, in einer Schichtstärke von mindestens 0,5 mm, insbesondere etwa 0,8 mm.

15. Dentalprothese nach einem der vorhergehenden Ansprüche, **dadurch gekennzeichnet, dass** der Zahnbogen (16) und die Prothesenbasis (12) je separat per Fräsen aus Rohlingen oder mittels additiven Verfahrens hergestellt sind und durch einen Kleber, insbesondere selbstpolymerisierendem Kleber, miteinander fest verbunden sind.

## Claims

1. A dental prosthesis, comprising a flesh-coloured prosthesis base (12) and a tooth-coloured tooth arrangement (14) which is partially embedded into the prosthesis base, wherein the dental prosthesis is configured as a hybrid prosthesis, wherein the prosthesis base thus has a contact surface which has a surface normal pointing towards the gums, wherein the tooth arrangement (14) surrounds a secondary part (22) or a primary part (50) on at least two sides (26, 28, 30) and is configured as a one-piece dental arch or as a segmented dental arch (16), **characterised in that** the secondary part or the primary part supports the tooth arrangement and a layer of prosthesis material of the prosthesis base lies between the tooth arrangement and the secondary part or primary part.

2. The dental prosthesis as claimed in claim 1, **characterised in that** the tooth-coloured tooth material of the tooth arrangement consists of a harder material than the prosthesis material of the prosthesis base (12).

3. The dental prosthesis as claimed in any one of the preceding claims, **characterised in that** the secondary part is configured as an external telescope and/or the primary part is configured as an abutment or as a tooth stump.

4. The dental prosthesis as claimed in any one of the preceding claims, **characterised in that** the tooth arrangement (14) is produced by a machining or constructive process, in particular is milled from a whole piece.

5. The dental prosthesis as claimed in any one of the preceding claims, **characterised in that** the tooth arrangement (14) surrounds, on its basal side, the external telescope or the primary part in the oral, vestibular and occlusal directions.

6. The dental prosthesis as claimed in any one of the preceding claims, **characterised in that** the tooth arrangement (14) forms, on its basal side and thus gum side, a bearing element which receives the secondary part or the primary part, optionally via a layer of prosthesis material of the prosthesis base.

7. The dental prosthesis as claimed in claim 6, **characterised in that** the bearing element of the tooth arrangement is embedded towards the outside, i.e. in the vestibular and oral directions, in prosthesis material of the prosthesis base (12) which supports the bearing element from the outside, and that the bearing element is exposed, i.e. is open, on the inside towards the gums.

8. The dental prosthesis as claimed in any one of the preceding claims, **characterised in that** the tooth arrangement (14) consists at least partially of highly cross-linked PMMA, in particular DCL material, and the prosthesis base (12) consists of less highly cross-linked PMMA.

9. The dental prosthesis as claimed in claim 6, **characterised in that** the tooth arrangement (14) is constructed of two layers or multiple layers of dentine material and of enamel material which is more translucent than the dentine material and that the dentine material extends in one piece to the bearing element and forms same.

10. The dental prosthesis as claimed in claim 6, **characterised in that** the tooth arrangement (14) has segments of at least 3 teeth which are fixedly connected to one another and to the bearing element via dentine material.

11. The dental prosthesis as claimed in any one of the preceding claims, **characterised in that** the dental prosthesis (10) is metal-free and is produced by CAD/CAM, in particular in machining or constructive processes.

12. The dental prosthesis as claimed in claim 6, **characterised in that** the bearing element is configured from tooth material in an arcuate manner in plan view, corresponding to the dental arch (16), and in a U-shape in cross-section, for the lower jaw prosthesis in the form of an upside-down U.

13. The dental prosthesis as claimed in any one of the preceding claims, **characterised in that** the tooth material extends to the contact surface and is covered by prosthesis material of the prosthesis base forming said contact surface.

14. The dental prosthesis as claimed in any one of the preceding claims, **characterised in that** the tooth material is covered by a layer of prosthesis material of the prosthesis base, in a layer thickness of at least 0.5 mm, in particular about 0.8 mm.

15. The dental prosthesis as claimed in any one of the preceding claims, **characterised in that** the dental arch (16) and the prosthesis base (12) are each produced separately by milling from blanks or by means of an additive process and are fixedly connected to one another by an adhesive, in particular a self-polymerising adhesive.

## Revendications

1. Prothèse dentaire, comprenant une base de prothèse de couleur chair (12) et un arrangement de dents (14) de la couleur des dents, qui est partiellement encastré dans la base de la prothèse, où la prothèse dentaire est conçue comme une prothèse hybride, c'est-à-dire où la base de la prothèse a une surface de contact qui a une normale de surface orientée vers l'extérieur, où l'arrangement de dents (14) entoure une partie secondaire (22) ou une partie primaire (50) sur au moins deux côtés (26, 28, 30) et est réalisé sous forme d'une arcade dentaire en un seule pièce ou d'une arcade dentaire segmentée (16) **caractérisé en ce que** la partie secondaire ou la partie primaire soutient l'arrangement de dents et qu'entre l'arrangement de dents et la partie secondaire ou primaire, il y a une couche de matériau prothétique à partir de la base de la prothèse.

2. Prothèse dentaire selon la revendication 1, **caractérisée en ce que** le matériau dentaire de la couleur des dents de l'arrangement de dents est constitué d'un matériau plus dur que le matériau de la prothèse de la base de la prothèse (12).

3. Prothèse dentaire selon l'une des revendications précédentes, **caractérisée en ce que** la partie secondaire est conçue comme un télescope externe et/ou que la partie primaire est formée comme un pilier ou comme un moignon de dent.

4. Prothèse dentaire selon l'une des revendications précédentes, **caractérisée en ce que** l'arrangement de dents (14) est fabriqué par un procédé ablatif ou structurant, en particulier est fraisé dans la masse.

5. Prothèse dentaire selon l'une des revendications précédentes, **caractérisée en ce que** l'arrangement de dents (14) entoure sur sa face basale le télescope externe ou la partie primaire au niveau oral, vestibulaire et occlusale.

6. Prothèse dentaire selon l'une des revendications précédentes, **caractérisée en ce que** l'agencement de dents (14) forme un élément de support sur sa face basale et donc gingivale, qui accueille la partie secondaire ou la partie primaire, le cas échéant par une couche de matériau prothétique de la base de la prothèse.

7. Prothèse dentaire selon la revendication 6, **caractérisée en ce que** l'élément de support de l'arrangement de dents est encastré vers l'extérieur, c'est-à-dire vestibulaire et oral, dans le matériau prothétique de la base de la prothèse (12), qui soutient l'élément de support de l'extérieur, et que l'élément de support est exposé vers l'intérieur de manière gingivale, c'est-à-dire ouvert.

8. Prothèse dentaire selon l'une des revendications précédentes, **caractérisée en ce que** l'arrangement de dents (14) est constitué, au moins en partie, de PMMA fortement réticulé, en particulier en matériau DCL, et la base de la prothèse (12) en PMMA moins fortement réticulé.

9. Prothèse dentaire selon la revendication 6, **caractérisée en ce que** l'arrangement de dents (14) est constitué de deux couches ou de plusieurs couches de matériau dentine et d'émail qui est plus translucide que le matériau dentine, et que le matériau dentine s'étend d'un seul tenant jusqu'à l'élément de support et forme celui-ci.

10. Prothèse dentaire selon la revendication 6, **caractérisée en ce que** l'arrangement de dents (14) comprend des segments d'au moins 3 dents qui sont solidement reliés entre eux et à l'élément de support par le biais du matériau dentine.

11. Prothèse dentaire selon l'une des revendications précédentes, **caractérisée en ce que** la prothèse dentaire (10) est sans métal et est fabriquée par CFAO, notamment selon des procédés ablatifs ou structurants.

12. Prothèse dentaire selon la revendication 6, **caractérisée en ce que** l'élément de support du matériau dentaire dans la vue de dessus est réalisé en forme d'arc correspondant à l'arcade dentaire (16), et en forme de U en coupe, pour la prothèse mandibulaire en forme de U renversé.

13. Prothèse dentaire selon l'une des revendications précédentes, **caractérisée en ce que** le matériau dentaire s'étend jusqu'à la surface d'appui et est recouvert par le matériau prothétique de la base de la prothèse qui forme celle-ci.

14. Prothèse dentaire selon l'une des revendications précédentes, **caractérisée en ce que** le matériau de la dent est recouvert d'une couche de matériau prothétique de la base de la prothèse, d'une épaisseur de couche d'au moins 0,5 mm, en particulier d'environ 0,8 mm.

15. Prothèse dentaire selon l'une des revendications précédentes, **caractérisée en ce que** l'arcade dentaire (16) et la base de la prothèse (12) sont fabriquées séparément par fraisage à partir de lingotins ou au moyen d'un procédé additif et sont solidement reliées entre elles par un adhésif, en particulier un adhésif autopolymérisant.
